Europäisches Patentamt

European Patent Office

Office européen des brevets

(19)

(11) Publication number: **0 000 432**
**B1**

## EUROPEAN PATENT SPECIFICATION

(12)

(45) Date of publication of patent specification: **25.05.83**

(21) Application number: **78300119.1**

(22) Date of filing: **05.07.78**

(51) Int. Cl.³: **C 07 C 15/02, C 07 C 5/22, B 01 J 29/28**

(54) **A process for effecting catalytic isomerization of monocyclic methyl-substituted aromatic hydrocarbon compounds.**

(30) Priority: **08.07.77 US 813911**

(43) Date of publication of application:
**24.01.79 Bulletin 79/2**

(45) Publication of the grant of the patent:
**25.05.83 Bulletin 83/21**

(84) Designated Contracting States:
**BE DE FR GB NL**

(56) References cited:
**DE - A - 2 558 035**
**US - A - 3 856 872**
**US - A - 3 992 466**

(73) Proprietor: **MOBIL OIL CORPORATION**
**150 East 42nd Street**
**New York New York 10017 (US)**

(72) Inventor: **Smith, Fritz Arthur**
**430 Hawthorne Avenue**
**Haddonfield New Jersey (US)**
Inventor: **Schwartz, Albert B.**
**1901 JFK,Blvd Apt 1204**
**Philadelphia Pennsylvania (US)**
Inventor: **Breckenridge,Lloyd Lee**
**1833 Lombard Street**
**Philadelphia Pennsylvania (US)**

(74) Representative: **Jones, Alan John et al,**
**CARPMAELS & RANSFORD 43 Bloomsbury Square**
**London, WC1A 2RA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

# 0 000 432

## A process for effecting catalytic isomerization of monocyclic methyl-substituted aromatic hydrocarbon compounds

The present invention relates to a process for the vapour-phase catalytic isomerization of monocyclic methyl-substituted aromatic hydrocarbon feedstock.

The catalytic rearrangement of alkyl groups present in alkyl aromatic hydrocarbons to provide one or more products, such as p-xylene, suitable for use in the petroleum and chemical industries has heretofore been effected by a wide variety of catalysts.

A recently developed and widely adopted catalyst for use in such processes is taught by U.S. Patent 3,856,872 to be of the ZSM-5 type of zeolite, whereby the process operates at high space velocities. Further, a process utilizing ZSM-5 type zeolites in acid form for vapor-phase conversion of a feedstock containing mixed $C_8$ aromatics in the absence of added hydrogen is taught by U.S. Patent 3,856,873. However, especially when the catalyst has increased acid activity, there can be a loss of xylene, the primary isomerization product, presumably due in part to disproportionation of xylenes and/or transalkylation of xylenes with any ethylbenzene which may be present in the reaction system.

The use of nitrogen compounds for various purposes in hydrocracking and reforming in the presence of catalyst other than that for use herein is known in the art. Examples of such art include U.S. Patents 2,849,377 (reforming); 3,657,110 (hydrocracking) and 3,694,345 (hydrocracking). Isomerization of alkyl aromatic compounds in the presence of nitrogen compounds to equilibrium products over catalyst other than that for use herein is taught in U.S. Patents 3,293,314 and 3,644,200. The catalyst for use in the process of U.S. Patent 3,293,314 is a solid acidic mixed metal-oxide catalyst, e.g., a silica-alumina mixture, having equilibration (tending to produce equilibrium products) and coke forming activities. Unfortunately, such catalyst materials lose their ability to maintain their equilibration tendency and, therefore, there is a decrease in desirable isomeric products as the catalysts age.

Our DTOS 2,558,035 discloses catalytic conversion of alkyl aromatic hydrocarbons in the presence of zeolitic catalysts. The conversion alternates between catalytic disproportionation of toluene and non-catalytic (i.e. thermal) dealkylation of toluene in the same reactor. The change from the catalytic to the non-catalytic conversion is effected by eliminating the acid activity of the catalyst by contacting it with a nitrogenous poison such as ammonia or an amine, and raising the temperature.

We have now found that if, in xylene isomerization, the acid activity of a zeolite catalyst is reduced by the use of a basic nitrogen compound and the conversion temperature raised to compensate for that reduction, the activity of the catalyst for xylene isomerization relative to its activity for xylene loss-promoting reactions will be increased.

According to the present invention a process for effecting catalytic isomerization of monocyclic methyl-substituted aromatic hydrocarbon compounds of from 8 to 10 carbon atoms contained in a feedstock also containing ethylbenzene comprises contacting said feedstock in the vapor phase in a reactor with a catalyst comprising a crystalline aluminosilicate zeolite having a constraint index within the range of 1 to 12, said zeolite containing hydrogen and/or Group VIII metal cations at a temperature of 316°C to 482°C (600°F to 900°F), a pressure of 101.3 kPa to 3548.7 kPa (0 psig to 500 psig), a hydrogen/hydrocarbon mole ratio of 0 to 10 and a weight hourly space velocity of 0.1 to 200, said catalyst having been contacted, in the reactor with one or more basic nitrogen compounds, the resulting ratio of nitrogen atoms/aluminum atoms in the zeolite being from 0.01 to 1.0, said nitrogen compounds being selected from ammonia, alkylamines of from 1 to 40 carbon atoms, alkylenediamines of from 2 to 40 carbon atoms, aromatic amines of from 6 to 40 carbon atoms, and heterocyclic nitrogen compounds.

The process of the present invention, wherein the catalyst has been contacted with one or more basic nitrogen-containing compounds, exhibits substantially reduced xylene loss. This reduction in xylene loss for isomerization of xylenes is an unexpected and significant benefit, and indicates that, whatever the monocyclic methyl-substituted aromatic hydrocarbon feedstock may be, substantial reduction in primary product losses will be effected and selectivity to desired product isomer will be enhanced.

The preferred zeolites for use in the process are zeolites ZSM-5, ZSM-11, ZSM-12, ZSM-35 and ZSM-38: they usually constitute from 10 to 90 weight percent of a composite with a binder therefor, such as alumina.

The preferred Group VIII metal cations are nickel, iron and/or cobalt. The process is particularly applicable to the isomerisation of a feedstock which contains xylenes.

The basic nitrogen containing compounds which are employed are ammonia, alkylamines of from 1 to 40 carbon atoms, alkylenediamines of from 2 to 40 carbon atoms, aromatic amines of from 6 to 40 carbon atoms e.g. aniline, and heterocyclic nitrogen compounds such as pyridine and pyrrolidine. Their contact with the catalyst may be effected by periodic or continuous addition to the feedstock.

Preferred zeolites for use in the present invention are those with a $SiO_2/Al_2O_3$ mole ratio of at least 12. Many are very well known. Zeolite ZSM-5, for instance, is described in U.S. Specification 3,702,886. Zeolite ZSM-11 is described in U.S. Specification 3,709,979. Zeolite ZSM-12 is described in U.S. Specification 3,970,544. Zeolite ZSM-35 is described in U.S. Specification 4,016,245. Zeolite

2

0 000 432

ZSM-38 is described in U.S. Specification 4,046,859. The significance and definition of "Constraint Index" is also well known from extensive patent literature such as DTOS 2,438,252.

In a preferred aspect of this invention, the catalysts hereof are selected as those having a crystal framework density, in the dry hydrogen form, of not substantially below 1.6 grams per cubic centimeter. It has been found that zeolites which satisfy all three of these criteria are most desired for the present process. Therefore, the preferred catalysts of this invention are those having a constraint index as defined above of 1 to 12, a silica to alumina ratio of at least 12 and a dried crystal density of not less than 1.6 grams per cubic centimeter. The dry density for known structures may be calculated from the number silicon plus aluminum atoms per 1000 cubic Angstroms ($1\text{Å}=10^{-10}\text{m}$), as given, e.g., on page 19 of the article on Zeolite Structure by W. M. Meier. This paper is included in "Proceedings of the Conference on Molecular Sieves, London, April 1967", published by the Society of Chemical Industry, London, 1968. When the crystal structure is unknown, the crystal framework density may be determined by classical pyknometer techniques. For example, it may be determined by immersing the dry hydrogen form of the zeolite in an organic solvent which is not sorbed by the crystal. It is possible that the unusual sustained activity and stability of this class of zeolite is associated with its high crystal anionic framework density of not less than 1.6 grams per cubic centimeter. This high density of course must be associated with a relatively small amount of free space within the crystal, which might be expected to result in more stable structures. This free space, however, is important as the locus of catalytic activity.

Crystal framework densities of some typical zeolites are:

| Zeolite | Void volume | Framework density |
|---|---|---|
| Ferrierite | 0.28 cc/cc | 1.76 g/cc |
| Mordenite | .28 | 1.7 |
| ZSM-5, —11 | .29 | 1.79 |
| Dachiardite | .32 | 1.72 |
| L | .32 | 1.61 |
| Clinoptilolite | .34 | 1.71 |
| Laumontite | .34 | 1.77 |
| ZSM-4 | .38 | 1.65 |
| Heulandite | .39 | 1.69 |
| P | .41 | 1.57 |
| Offretite | .40 | 1.55 |
| Levynite | .40 | 1.54 |
| Erionite | .35 | 1.51 |
| Gmelinite | .44 | 1.46 |
| Chabazite | .47 | 1.45 |
| A | .5 | 1.3 |
| Y | .48 | 1.27 |

The amount of ethylbenzene in the feedstock is usually from 5 to 30 weight percent, more usually from 10 to 25 weight percent. Specific compounds contemplated for isomerisation include para-xylene, meta-xylene, ortho-xylene, mesitylene (1,3,5-trimethylbenzene) durene (1,2,4,5-tetramethylbenzene), hemimellitene (1,2,3-trimethylbenzene), pseudocumene (1,2,4-trimethylbenzene), prehnitene (1,2,3,4-tetramethylbenzene) and isodurene (1,2,3,5-tetramethylbenzene) with the xylene isomers and pseudocumene especially preferred.

The nitrogen-containing compounds useful in the present process are ones which neither react with the charge materials nor possess catalytic activity contrary to the purpose for their use. Said nitrogen-containing compounds may be gaseous, liquid or in the form of a solid dissolved in a suitable solvent, such as, for example, toluene.

The nitrogen compounds useful in this invention are ammonia, alkylamines of from 1 to about 40 carbon atoms, and preferably from 1 to 10 carbon atoms, e.g. n-propylamine, alkylenediamines of from 2 to 40 carbon atoms, and preferably from 6 to 20 carbon atoms, aromatic amines of from 6 to 40 carbon atoms, e.g. aniline, and heterocyclic nitrogen compounds, such as pyridine and pyrrolidine.

The catalyst may be contacted in the reactor with the nitrogen compound in a variety of ways. For example, the nitrogen compound can be contacted with the catalyst by adding it to the feedstock periodically or continuously.

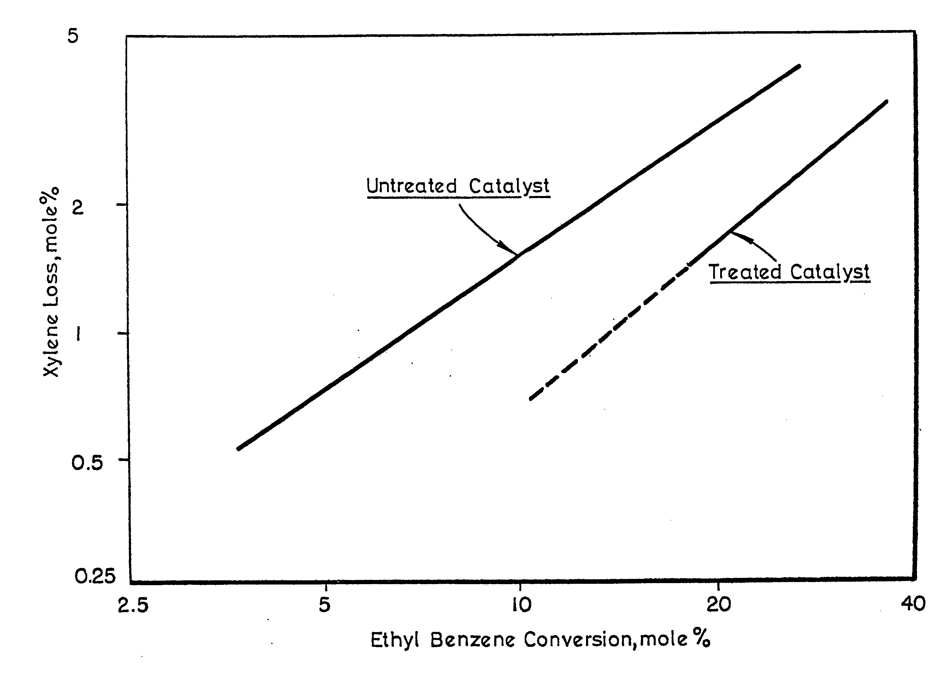

The specific examples which follow illustrate, in Example 1 the preparation of a zeolite for use in the later Examples, and in Examples 2 to 6 embodiments of the process of the present invention, as well as comparative procedures. The single Figure of the Drawing summarises the data of Examples 2 to 4.

Example 1

A sodium silicate solution was prepared by mixing 16 parts water and 27.7 parts sodium silicate (28.7 wt.% $SiO_2$, 8.9 wt.% $Na_2O$, 62.4% $H_2O$). The solution was cooled to approximately 15°C.

3

**0 000 432**

An acid solution was prepared by adding 1 part aluminum sulfate (17.2 wt.% $Al_2O_3$) to 16.4 parts water followed by 2.4 parts sulfuric acid (93 wt.% $H_2SO_4$) and 1.2 parts NaCl.

These solutions were mixed in an agitated vessel while 3.9 parts of NaCl were added. The gel molar ratios expressed as oxides are the following:

$$SiO_2/Al_2O_3 = 78.4$$

$$Na_2O/Al_2O_3 = 49.9$$

An organic solution was prepared by adding 1.6 parts n-propyl bromide and 3.1 parts methyl ethyl ketone to 1.9 parts tri-n-propylamine.

After the gel was heated to about 95°C, agitation was reduced and the organic solution was added above the gel. This mixture was held at 95—110°C for 14 hours, then agitation was increased. When approximately 65% of gel was crystallized the temperature was increased to 150—160°C and held there until crystallization was complete. Unreacted organics were removed by flashing and the remaining contents cooled.

The zeolite slurry product was diluted with 4—5 parts water per part slurry, allowed to settle and supernatent liquid was drawn off. The settled solids were reslurried to the original volume of the preceding step with water. After settling, the aqueous phase was decanted. This procedure was repeated until the sodium level of the zeolite was less than 1.0 wt.%. The washed zeolite was then filtered, dried and identified as ZSM-5 having a silica/alumina mole ratio of about 70 and a constraint index of about 8.3.

The dried zeolite was then mixed with alumina and water. It was then extruded into 2.9 cm (1.16″) pellets and dried. The extruded material contained 65 parts ZSM-5 per 35 parts alumina, by weight.

The dried extrudate was calcined for three hours at 538°C in flowing nitrogen. After cooling, the extrudate was contacted with an ammonium nitrate exchange solution (about 0.08 kg $NH_4NO_3$/kg extrudate) for one hour at ambient temperature. This exchange was then repeated until the sodium level was less than 0.05 wt.%. The extrudate was then contacted with a nickel nitrate exchange solution (about 0.1 kg Ni ($Ni(NO_3)_2 \cdot 6H_2O$/kg extrudate) for two hours at about 80—90°C. After this exchange, the extrudate was washed, dried and calcined in a flowing 10% air-90% nitrogen mixture at 538°C for six hours.

Example 2

25 cc of the catalyst of Example 1 was further calcined at 538°C (1000°F) in air for 24 hours and placed in an isothermal, one gallon/day reactor. Pure hydrogen was continuously passed through the reactor without recycle in order to maintain a constant molar ratio between hydrogen and hydrocarbon feedstock at the reactor inlet. A feedstock comprising the components listed in Table 2 was then passed through the reactor under the conditions listed in Table 3, hereinafter presented. Also listed in Table 3 are the results of ten separate material balances over the period of 10.5 days on stream. It will be noted that from the data generated at each material balance, an approach to equilibrium was calculated as being (weight % p-or o-isomer, respectively, of total xylenes product—weight % of that isomer in the feed) divided by (equilibrium weight % concentration of p- or o-isomer, respectively at the reaction temperature—weight % of that isomer in feed). The p- or o-isomer equilibrium concentrations, respectively, as a function of reaction temperature may be determined according to *The Chemical Thermodynamics of Organic Compounds* by Stull, Westrum and Sinke, pulbished in 1969 by Wiley.

For the improved isomerization process of this invention, the zeolite is preferably employed in association with a support or binder material which acts as diluent such as, for example, a porous inorganic oxide support or a clay binder. Non-limiting examples of such binder materials include alumina, zirconia, silica, magnesia, thoria, titania, boria and combinations thereof, generally in the form of dried inorganic oxide gels and gelatinous precipitates. Suitable clay materials include, by way of example, bentonite and kieselguhr. The relative proportion of suitable crystalline aluminosilicate zeolite of the total composition of catalyst and binder or support may vary with the zeolite content ranging from between 10 to 90 percent by weight and more usually in the range of 20 to 80 percent by weight of the composition.

Operating conditions employed in the improved process of the present invention are important. Such conditions as temperature, pressure, space velocity, molar ratio of the reactants, hydrogen to hydrocarbon mole ratio, and the presence of any feedstock diluents, such as toluene and/or $C_9^+$ recycle material, will have important effects on the process. Fixed or fluid bed contacting may be employed. Weight hourly space velocity (WHSV) is the weight of charge which contacts unit weight of zeolite per hour.

Within the limits of operating conditions hereinbefore set forth, the conditions of temperature and pressure adopted in practice will vary considerably depending upon equilibrium considerations and type of feed material. Optimum conditions are those in which maximum yields of desired isomer products are obtained and hence considerations of temperature and pressure will vary within a range of conversion levels designed to provide the highest selectivity and maximum yield.

4

TABLE 2
Feedstock composition for example 2

| Component | Amount, wt.% |
| --- | --- |
| Toluene | 0.04 |
| Ethylbenzene | 14.81 |
| p-Xylene | 10.07 |
| m-Xylene | 59.29 |
| o-Xylene | 15.69 |
| Cg+ Aromatics | 0.11 |

# TABLE 3
## Example 2—reaction conditions and results

| Material Balance | 1 | 2 | 3 | 4 | 5 | 6 | 7 | 8 | 9 | 10 |
|---|---|---|---|---|---|---|---|---|---|---|
| Days on Stream | 0.8 | 1.9 | 2.9 | 4.8 | 5.5 | 6.5 | 7.5 | 8.5 | 9.5 | 10.5 |
| Temperature, °C (°F) | 283(541) | 283(542) | 294(561) | 293(560) | 305(581) | 305(581) | 316(601) | 316(601) | 327(621) | 327(620) |
| WHSV, hr.$^{-1}$ | 7.14 | 7.21 | 7.25 | 7.20 | 7.26 | 7.17 | 7.25 | 7.25 | 7.20 | 7.19 |
| Pressure, kPa (psig) | ← | | | | 1480(200) | | | | | → |
| H2/HC Ratio | 4.07 | 4.00 | 3.98 | 4.05 | 4.02 | 4.03 | 3.99 | 3.99 | 3.78 | 3.96 |
| Ethylbenzene | | | | | | | | | | |
|   Conversion, mole % | 3.63 | 3.69 | 6.58 | 6.64 | 11.19 | 11.77 | 17.95 | 18.40 | 26.53 | 26.77 |
| Xylene Loss, mole % | .46 | .49 | .92 | 1.11 | 1.69 | 1.73 | 2.77 | 2.63 | 3.92 | 3.95 |
| Approach to Equilibrium | | | | | | | | | | |
|   for p-Xylene | 97.4 | 97.4 | 99.8 | 100.4 | 100.5 | 100.5 | 101.4 | 101.1 | 100.6 | 101.5 |
| Approach to Equilibrium | | | | | | | | | | |
|   for o-Xylene | 60.8 | 65.4 | 73.1 | 72.1 | 77.7 | 77.1 | 79.9 | 79.8 | 81.1 | 79.8 |
| Yields, mole % | | | | | | | | | | |
|   Benzene | .43 | .43 | .75 | .77 | 1.32 | 1.32 | 2.22 | 2.16 | 3.35 | 3.31 |
|   Toluene | .30 | .33 | .45 | .50 | .68 | .68 | .88 | .86 | 1.16 | 1.16 |
|   Ethylbenzene | 14.27 | 14.26 | 13.84 | 13.83 | 13.15 | 13.07 | 12.15 | 12.09 | 10.88 | 10.85 |
|   p-Xylene | 20.08 | 20.07 | 20.19 | 20.21 | 20.05 | 20.05 | 19.88 | 19.88 | 19.52 | 19.60 |
|   m-Xylene | 47.24 | 47.10 | 46.32 | 46.21 | 45.65 | 45.64 | 44.87 | 44.97 | 44.28 | 44.23 |
|   o-Xylene | 17.33 | 17.46 | 17.75 | 17.68 | 17.91 | 17.88 | 17.94 | 17.96 | 17.91 | 17.85 |
|   Methylethylbenzene | .06 | .08 | .10 | .13 | .17 | .18 | .22 | .22 | .28 | .29 |
|   Trimethylbenzene | .09 | .08 | .17 | .18 | .23 | .26 | .35 | .35 | .45 | .47 |
|   Diethylbenzene | .11 | .11 | .22 | .24 | .40 | .42 | .63 | .65 | .83 | .86 |
|   Dimethylethylbenzene | .08 | .09 | .21 | .25 | .45 | .51 | .84 | .87 | 1.33 | 1.39 |

Example 3

After 10.5 days on stream, the catalyst used in Example 2 was contacted in the same reactor as for Example 2 with a feedstock containing a nitrogen containing compound, i.e., 1.10-decane diamine as indicated in Table 4, hereinafter presented. The feedstock containing the nitrogen-containing compound was pumped through the reactor for 24 hours at operating conditions of 316°C (600°F), 1480 kPa (200 psig) a WHSV of 7 hr$^{-1}$ and a hydrogen/hydrocarbon mole ratio of 4. The ratio of nitrogen atoms provided in the feed per aluminum atom in the zeolite component was 3.4.

TABLE 4

Feedstock composition for example 3 containing a nitrogen-containing compound

| Component | Amount |
|---|---|
| 1,10-Decane diamine | 500 ppm |
| Toluene | 0.04 wt % |
| Ethylbenzene | 15.60 wt % |
| p-Xylene | 11.53 wt % |
| m-Xylene | 57.72 wt % |
| o-Xylene | 15.02 wt % |
| $C_9^+$ Aromatics | 0.09 wt T |

Thereafter, the above feedstock without the nitrogen-containing compound was charged to the reactor and at the reaction conditions of Example 2, the catalyst was found to be inactive. The reaction temperature was increased, through increments, up to about 399°C (750°F), where a rapid reactivation of the catalyst was observed, as a result of desorption of part of the nitrogen from the catalyst, bringing the ratio of nitrogen atoms/aluminum atom in the zeolite component within the range of 0.01 to 1.0. The temperature was then reduced to 371°C (700°F) where gradual recovery of catalyst activity was still observable. The reaction conditions and results from 8 material balances over the period of about 8 days on stream appear in Table 5.

TABLE 5
Example 3—reaction conditions and results

| Material Balance | 11 | 12 | 13 | 14 | 15 | 16 | 17 | 18 |
|---|---|---|---|---|---|---|---|---|
| Days on Stream | 24.3 | 25.2 | 26.2 | 27.1 | 28.2 | 29.8 | 31.1 | 32.1 |
| Temperature, °C (°F) | 316(600) | 316(600) | 343(650) | 371(700) | 400(752) | 399(751) | 399(750) | 371(700) |
| WHSV, hr.$^{-1}$ | 7.19 | 7.14 | 7.18 | 7.18 | 7.24 | 7.22 | 7.12 | 7.31 |
| Pressure, kPa (psig) | | | | ←————1480(200)————→ | | | | |
| H2/HC Ratio | 4.01 | 4.04 | 4.00 | 3.98 | 3.95 | 4.00 | 4.03 | 3.95 |
| Ethylbenzene | | | | | | | | |
| Conversion, mole % | .00 | −.18 | 1.02 | 1.18 | 19.95 | 33.71 | 36.57 | 20.35 |
| Xylene Loss, mole % | .11 | .07 | −.21 | −.16 | 1.58 | 2.91 | 3.68 | 1.89 |
| Activity Loss, °F | 179 | | 159 | 205 | 149 | 122 | 117 | 94 |
| Approach to Equilibrium for p-Xylene | .6 | .5 | 2.6 | 30.6 | 101.3 | 101.8 | 102.4 | 100.9 |
| Approach to Equilibrium for o-Xylene | .8 | −.2 | −3.9 | 21.6 | 80.1 | 80.1 | 82.7 | 82.3 |
| Yields, mole % | | | | | | | | |
| Benzene | .00 | .00 | .00 | .10 | 2.84 | 4.74 | 5.30 | 2.78 |
| Toluene | .12 | .06 | .03 | .04 | .28 | .55 | .71 | .33 |
| Ethylbenzene | 15.60 | 15.63 | 15.44 | 15.42 | 12.49 | 10.34 | 9.89 | 12.43 |
| Paraxylene | 11.57 | 11.57 | 11.78 | 14.12 | 19.62 | 19.39 | 19.29 | 19.61 |
| Metaxylene | 57.58 | 57.64 | 57.79 | 54.19 | 44.42 | 43.70 | 43.26 | 44.43 |
| Orthoxylene | 15.04 | 15.00 | 14.88 | 16.09 | 18.90 | 18.64 | 18.62 | 18.63 |
| Propylbenzene | .08 | .08 | .07 | .04 | .00 | .00 | .00 | .00 |
| Methylethylbenzene | .00 | .00 | .00 | .00 | .05 | .12 | .17 | .07 |
| Trimethylbenzene | .02 | .02 | .01 | .00 | .08 | .17 | .21 | .08 |
| Diethylbenzene | .00 | .00 | .00 | .01 | .65 | .97 | 1.02 | .80 |
| Dimethylethylbenzene | .00 | .00 | .00 | .00 | .67 | 1.29 | 1.53 | .84 |

Example 4

The experiment of Example 3 was continued with a feedstock composed as indicated in Table 6. The conditions for and results from 10 material balances over a period of about 10 days on stream appear in Table 7.

TABLE 6
Feedstock composition for example 4

| Component | Amount, wt.% |
|---|---|
| Toluene | 0.04 |
| Ethylbenzene | 15.20 |
| p-Xylene | 10.26 |
| m-Xylene | 59.86 |
| o-Xylene | 14.55 |
| $C_9^+$ Aromatics | 0.08 |

## TABLE 7
### Example 4—reaction conditions and results

| Material Balance | 19 | 20 | 21 | 22 | 23 | 24 | 25 | 26 | 27 | 28 |
|---|---|---|---|---|---|---|---|---|---|---|
| Days on Stream | 34.1 | 36.1 | 37.1 | 38.1 | 39.1 | 40.1 | 41.1 | 42.1 | 43.1 | 44.8 |
| Temperature, °C (°F) | 371(700) | 372(701) | 372(701) | 372(701) | 371(700) | 371(700) | 371(700) | 371(700) | 371(700) | 371(700) |
| WHSV, hr.$^{-1}$ | 7.19 | 7.22 | 7.22 | 7.21 | 7.22 | 7.22 | 7.25 | 7.14 | 7.12 | 7.19 |
| Pressure, kPa (psig) | ← | | | | 1480(200) | | | | | → |
| H2/HC Ratio | 3.97 | 3.86 | 3.92 | 3.93 | 3.93 | 3.93 | 3.91 | 3.98 | 3.97 | 3.94 |
| **Ethylbenzene** | | | | | | | | | | |
| Conversion, mole % | 20.41 | 21.75 | 22.42 | 23.04 | 23.32 | 23.49 | 23.93 | 24.12 | 24.88 | 25.34 |
| Xylene Loss, mole % | 1.86 | 1.84 | 1.99 | 1.92 | 2.00 | 1.88 | 2.03 | 2.13 | 2.01 | 2.15 |
| Activity Loss, °F | 96 | 94 | 92 | 91 | 90 | 89 | 89 | 88 | 87 | 86 |
| Approach to Equilibrium for p-Xylene | 101.1 | 100.8 | 101.2 | 100.7 | 100.9 | 100.3 | 100.9 | 101.6 | 100.5 | 100.9 |
| Approach to Equilibrium for o-Xylene | 81.4 | 81.5 | 81.1 | 80.1 | 76.5 | 84.9 | 82.7 | 82.8 | 97.1 | 78.6 |
| **Yields, mole %** | | | | | | | | | | |
| Benzene | 2.67 | 2.84 | 2.96 | 3.03 | 3.00 | 3.04 | 3.17 | 3.10 | 3.19 | 3.43 |
| Toluene | .33 | .35 | .36 | .36 | .36 | .37 | .38 | .41 | .40 | .42 |
| Ethylbenzene | 12.10 | 11.90 | 11.79 | 11.70 | 11.66 | 11.63 | 11.57 | 11.54 | 11.42 | 11.35 |
| Paraxylene | 19.75 | 19.72 | 19.72 | 19.69 | 19.70 | 19.66 | 19.69 | 19.74 | 19.66 | 19.67 |
| Metaxylene | 44.77 | 44.79 | 44.72 | 44.84 | 44.98 | 44.65 | 44.64 | 44.52 | 44.88 | 44.80 |
| Orthoxylene | 18.59 | 18.60 | 18.55 | 18.51 | 18.30 | 18.77 | 18.62 | 18.61 | 18.43 | 18.38 |
| Methylethylbenzene | .07 | .07 | .07 | .07 | .07 | .07 | .08 | .08 | .08 | .08 |
| Trimethylbenzene | .09 | .09 | .10 | .09 | .16 | .10 | .10 | .11 | .10 | .11 |
| Diethylbenzene | .77 | .78 | .83 | .81 | .84 | .83 | .83 | .90 | .85 | .85 |
| Dimethylethylbenzene | .86 | .85 | .89 | .90 | .93 | .88 | .92 | .99 | .99 | .92 |

**0 000 432**

In the single figure of the drawing ethylbenzene conversion is plotted against xylene loss for the results of Example 2, Example 3 and Example 4. It is readily observed that at a particular ethylbenzene conversion, the process of the present invention, wherein the catalyst has been contacted with a nitrogen-containing compound, exhibits substantially reduced xylene loss. This reduction in xylene loss for isomerization of xylenes is an unexpected and significant benefit and indicates that, whatever the monocyclic methyl-substituted aromatic hydrocarbon feedstock may be, substantial reduction in primary product losses will be effected and selectivity to desired product isomer will be enhanced.

Example 5

A catalyst prepared as in Example 1, but not contacted with the nickel nitrate solution, was placed in the reactor used in Examples 2—4. A feedstock composed as described in Table 8 was then passed through the reactor at 273.7 kPa (25 psig), 316°C (600°F), a WHSV of 5 hr⁻¹ and a hydrogen/hydrocarbon mole ratio of 0 (absence of added hydrogen). The results of this experiment are presented in Table 9.

Example 6

The feedstock for Example 5 is then injected with ammonia to the extent that the ratio of nitrogen atoms/aluminum atom in the zeolite is 0.1. The injection of ammonia into the feedstock is stopped after a time and the reaction temperature is increased to 399°C (750°F). Results of this experiment are also presented in Table 9 for comparison with the results from Example 5.

### TABLE 8
#### Feedstock composition for example 5

| Component | Amount. wt.% |
|---|---|
| Toluene | 0.9 |
| Ethylbenzene | 6.9 |
| p-Xylene | 10.5 |
| m-Xylene | 62.9 |
| o-Xylene | 18.7 |
| $C_9^+$ Aromatics | — |

### TABLE 9
#### Results from examples 5 and 6

| Example | 5 | 6 |
|---|---|---|
| Ethylbenzene Conversion, Wt. % | 24.6 | 24.6 |
| Xylene Loss, Wt. % | 1.84 | 0.87 |
| Approach to Equilibrium for p-xylene | 102.8 | 102.0 |
| Yield, Wt. % | | |
| —$C_1$—$C_5$ hydrocarbons | — | 0.1 |
| Benzene | 0.7 | 1.2 |
| Toluene | 1.7 | 1.1 |
| Ethylbenzene | 5.2 | 5.2 |
| p-Xylene | 21.9 | 21.7 |
| m-Xylene | 49.0 | 48.6 |
| o-Xylene | 19.5 | 21.0 |
| $C_9^+$ Aromatics | 2.0 | 1.1 |

## Claims

1. A process for effecting catalytic isomerization of monocyclic methyl-substituted aromatic hydrocarbon compounds of from 8 to 10 carbon atoms contained in a feedstock also containing ethylbenzene which comprises contacting said feedstock in the vapor phase in a reactor with a catalyst comprising a crystalline aluminosilicate zeolite having a constraint index within the range of 1 to 12, said zeolite containing hydrogen and/or Group VIII metal cations, at a temperature of 316°C to 482°C (600°F to 900°F), a pressure of 101.3 kPa to 3548.7 kPa (0 psig to 500 psig), a hydrogen/hydrocarbon mole ratio of 0 to 10 and a weight hourly space velocity of 0.1 to 200, said catalyst having been contacted in the reactor, with one or more basic nitrogen compounds, the resulting ratio of nitrogen atoms/aluminum atoms in the zeolite being from 0.01 to 1.0, said nitrogen compounds being selected from ammonia, alkylamines of from 1 to 40 carbon atoms, alkylenediamines of from 2 to 40 carbon atoms, aromatic amines of from 6 to 40 carbon atoms, and heterocyclic nitrogen compounds.

11

# 0 000 432

2. A process according to Claim 1 wherein said zeolite is ZSM-5, ZSM-11, ZSM-12, ZSM-35 or ZSM-38.

3. A process according to Claim 1 or Claim 2 wherein said zeolite constitutes from 10 to 90 weight percent of a composite with a binder therefor.

4. A process according to Claim 3 wherein said binder is alumina.

5. A process according to any preceding claim wherein said Group VIII metal cations are nickel, iron and/or cobalt.

6. A process according to any preceding claim wherein said feedstock contains xylenes.

7. A process according to any of Claims 1 to 6 wherein the contact of the nitrogen compound with the catalyst is effected by periodic or continuous addition of that compound to the feedstock.


**Patentansprüche**

1. Verfahren zur Ausführung von katalytischer Isomerisierung von monocyclischen methylsubstituierten aromatischen Kohlenwasserstoffverbindungen mit 8 bis 10 Kohlenstoffatomen, enthalten in einem Einsatzmaterial, welches ferner Äthylbenzol enthält, dadurch gekennzeichnet, daß man das Einsatzmaterial in der Dampfphase in einem Reaktor mit einem Katalysator, enthaltend einen kristallinen Aluminosilikat-Zeolithen mit einem Zwangsindex innerhalb des Bereiches von 1 bis 12, wobei der Zeolith Wasserstoff und/oder Gruppe VIII-Metallkationen enthält, bei einer Temperatur von 316°C bis 482°C (600°F bis 900°F), einem Druck von 101,3 kPa bis 3548,7 kPa (0 psig bis 500 psig), einem Wasserstoff/Kohlenwasserstoff-Molverhältnis von 0 bis 10 und einer gewichtsmäßigen stündlichen Raumströmungsgeschwindigkeit von 0,1 bis 200 in Berührung bringt, wobei der Katalysator im Reaktor mit einer oder mehreren basischen Stickstoffverbindungen in Berührung gebracht worden ist, das resultierende Verhältnis von Stickstoffatomen/Aluminiumatomen im Zeolithen von 0,01 bis 1,0 geht und die Stickstoffverbindungen aus der Reihe von Ammoniak, Alkylaminen mit 1 bis 40 Kohlenstoffatomen, Alkylendiaminen mit 2 bis 40 Kohlenstoffatomen, aromatischen Aminen mit 6 bis 40 Kohlenstoffatomen und heterocyclischen Stickstoffverbindungen ausgewählt sind.

2. Verfahren nach Anspruch 1, dadurch gekennzeichnet, daß der Zeolith ZSM-5, ZSM-11, ZSM-12, ZSM-35 oder ZSM-38 ist.

3. Verfahren nach Anspruch 1 oder 2, dadurch gekennzeichnet, daß der Zeolith 10 bis 90 Gew.-% der Mischung mit einem Bindemittel dafür darstellt.

4. Verfahren nach Anspruch 3, dadurch gekennzeichnet, daß das Bindemittel Aluminiumoxid ist.

5. Verfahren nach einem der vorhergehenden Ansprüche, dadurch gekennzeichnet, daß die Gruppe VIII-Metallkationen Nickel, Eisen und/oder Kobalt sind.

6. Verfahren nach einem der vorhergehenden Ansprüche, dadurch gekennzeichnet, daß das Einsatzmaterial Xylole enthält.

7. Verfahren nach einem der Ansprüche 1 bis 6, dadurch gekennzeichnet, daß die Berührung der Stickstoffverbindung mit dem Katalysator durch periodische oder kontinuierliche Zugabe der betreffenden Verbindung zum Einsatzmaterial ausgeführt wird.


**Revendications**

1. Procédé pour effectuer l'isomérisation catalytique de composés hydrocarbonés aromatiques, monocycliques, substitués par des groupes méthyle, ayant de 8 à 10 atomes de carbone, contenus dans une matière première (charge) contenant également de l'éthylbenzène, caractérisé en ce qu'il consiste à mettre ladite charge en contact, en phase vapeur, dans un réacteur, avec un catalyseur comprenant une zéolite d'aluminosilicate cristalline ayant un indice de contrainte dans la gamme de 1 à 12, ladite zéolite contenant de l'hydrogéne et/ou des cations d'un métal du Groupe VIII, à une température de 316 à 482°C, une pression manométrique de 101,3 kPa à 3548,7 kPa, un rapport molaire hydrogène/hydrocarbure de 0 à 10 et une vitesse horaire spatiale en poids de 0,1 à 200, ledit catalyseur ayant été mis en contact, dans le réacteur, avec un ou plusieurs composés d'azote basiques, le rapport atomes d'azote/atomes d'aluminium résultant dans la zéolite étant de 0,01 à 1,0, lesdits composés d'azote étant choisis parmi l'ammoniac, des alkylamines ayant de 1 à 40 atomes de carbone, des alkylènediamines ayant de 2 à 40 atomes de carbone, des amines aromatiques ayant de 6 à 40 atomes de carbone et des composés d'azote hétérocycliques.

2. Procédé selon la revendication 1, caractérisé en ce que ladite zéolite est l'une des zéolites suivantes: ZSM-5, ZSM-11, ZSM-12, ZSM-35 ou ZSM-38.

3. Procédé selon l'une des revendications 1 ou 2, caractérisé en ce que la zéolite constitue de 10 à 90% en poids d'un composite contenant également un liant.

4. Procédé selon la revendication 3, caractérisé en ce que ledit liant est l'alumine.

5. Procédé selon l'une quelconque des revendications précédentes, caractérisé en ce que les cations du métal du Groupe VIII sont le nickel, le fer et/ou le cobalt.

6. Procédé selon l'une quelconque des revendications précédentes, caractérisé en ce que la charge contient des xylènes.

12

7. Procédé selon l'une quelconque des revendications 1 à 6, caractérisé en ce que le contact du composé azoté avec le catalyseur est réalisé par une addition périodique ou continue de ce composé à la charge.